(19) Europäisches Patentamt
European Patent Office
Office européen des brevets

(11) Veröffentlichungsnummer : **0 042 352**
**B1**

(12) # EUROPÄISCHE PATENTSCHRIFT

(45) Veröffentlichungstag der Patentschrift : **28.12.83**

(51) Int. Cl.³ : **C 07 C 101/18**, C 07 C 99/00

(21) Anmeldenummer : **81810216.2**

(22) Anmeldetag : **03.06.81**

(54) **Verfahren zur Herstellung von N-(1'-Alkoxycarbonyläthyl)-2,6-dialkylanilinen.**

(30) Priorität : **09.06.80 US 157761**

(43) Veröffentlichungstag der Anmeldung : **23.12.81 Patentblatt 81/51**

(45) Bekanntmachung des Hinweises auf die Patenterteilung : **28.12.83 Patentblatt 83/52**

(84) Benannte Vertragsstaaten : **AT BE CH DE FR GB IT LI NL SE**

(73) Patentinhaber : **CIBA-GEIGY AG**
**Patentabteilung Postfach**
**CH-4002 Basel (CH)**

(72) Erfinder : **Grieder, Alfred, Dr.**
**Ob den Reben 15**
**CH-4461 Böckten (CH)**
Erfinder : **Coers, Klaus Jürgen**
**Lochackerstrasse 10**
**CH-4153 Reinach (CH)**

(56) Entgegenhaltungen :
**EP-A- 0 008 057**
**DE-A- 2 153 356**
**DE-A- 2 812 169**
**US-A- 4 025 648**
**ACTA CHEMICA SCANDINAVICA B, 29 (1975) no. 2, Copenhagen, DK A. BRANDSTROM et al. "Ion pair extraction in preparative organic chemistry, IX. Kinetic evidence for an ion pair mechanism in the halogen exchange of alkyl halides catalyzed by tetrabutylammonium halides" Seiten 201-205**
**JOURNAL OF THE AMERICAN CHEMICAL SOCIETY, Band 92(1), 13, Januar 1971 Washington, US C.M. STARKS "Phase-transfer catalysis. I. Heterogeneous reactions involving anion transfer by quaternary ammonium and phosphonium salts" Seiten 195-199**
**METHODEN DER ORGANISCHEN CHEMIE, Houben-Weyl, 4e Aufl. Herausgegeben von Eugen Müller, Band XI/1 : "Stickstoffverbindungen II" 1957, G. Thieme Verlag, Stuttgart, DE G. SPIELBERGER : "Amine durch Austauschreaktionen", Seite 30.**
**METHODEN DER ORGANISCHEN CHEMIE, Houben-Weyl, 4e Aufl. Herausgegeben von Eugen Müller,**

(56) Entgegenhaltungen :
**Band XI/2 « Stickstoffverbindungen II (Fortsetzung) ; Stickstoffverbindungen III », 1958, Thieme Verlag, Stuttgart, DE Th. WIELAND et al. « Methoden zur Herstellung und Umwandlung von Aminosäuren und Derivaten ». Seiten 309-310**
**SYNTHESIS, Juni 1976, Stuttgart, DE V. BOCCHI et al. « Synthesis of N-alkylindoles using tetraalkylammonium salt catalysis » Seiten 414-416**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 042 352 B1

Verfahren zur Herstellung von N-(1'-Alkoxycarbonyläthyl)-2,6-dialkylanilinen

Die vorliegende Erfindung bezieht sich auf ein Verfahren zur Herstellung von N-(1'-Alkoxycarbonyläthyl)-2,6-dialkylanilinen der Formel I

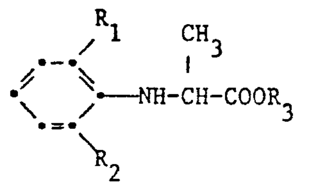

(I)

in welcher $R_1$ und $R_2$ je Methyl oder Aethyl bedeuten und $R_3$ für eine Alkylgruppe mit 1-4 Kohlenstoffatomen steht.

Die N-(1'-Alkoxycarbonyläthyl)-2,6-dialkylaniline der Formel I sind wertvolle Zwischenprodukte für die Herstellung von pestizid wirksamen Verbindungen. Sie können beispielsweise durch Umsetzung mit Säurechloriden, wie Chloracetylchlorid, Methoxyacetylchlorid oder Furan-2-carbonsäurechlorid in entsprechende N-Acylamine übergeführt werden, die sich durch eine hervorragende Wirkung gegen phytopatogene Mikroorganismen auszeichnen und daher ausgedehnte Anwendung im Pflanzenschutz finden. Solche N-Acylaniline, sowie ihre Herstellung und Verwendung sind beispielsweise in den US-Patentschriften 4,008,066, 4,094,990 und 4,151,299 beschrieben.

Unter den N-Acylanilinen der vorgenannten Art, die durch Acylierung der verbindungsgemäss herstellbaren N-(1'-Alkoxycarbonyläthyl)-2,6-dialkylaniline der Formel I hergestellt werden können, sind insbesondere das N-Methoxyacetyl-N-(1'-methoxycarbonyläthyl)-2,6-dimethylanilin und das N-(2''-Furoyl)-N-(1'-methoxycarbonyläthyl)-2,6-dimethylanilin zu nennen.

Es ist bekannt, N-(1'-Alkoxycarbonyläthyl)-2,6-dialkylaniline durch Umsetzung von 2,6-Dialkylanilinen mit α-Halogenpropionsäureestern herzustellen. Diese Umsetzung ist einerseits wegen der durch die orthoständigen Alkylgruppen bedingten sterischen Hinderung und andererseits wegen der Hydrolyseempfindlichkeit der α-Halogenpropionsäureester problematisch. Zur Lösung der mit der Durchführung dieses Verfahrens verbundenen Probleme wurden bereits verschiedene Vorschläge gemacht.

So ist in der US-Patentschrift 3,882,162 die Umsetzung von α-Halogenessigsäureestern und 2,6-Dialkylanilinen in Gegenwart von wässrigen Alkalmetallhydroxiden erwähnt. Nach dieser Methode werden die gewünschten Produkte infolge von Nebenreaktionen, wie N,N-dialkylierung und Hydrolyse der Estergruppe, nur in ungenügender Reinheit und in unbefriedigenden Ausbeuten erhalten.

Gemäss US-Patentschrift 3,882,162 wird vorgeschlagen, die mit dem vorgenannten Verfahren verbundenen Nachteile dadurch zu vermeiden, dass man die Umsetzung des 2,6-Dialkylanilins mit dem α-Halogencarbonsäureester in Gegenwart von überschüssigem 2,6-Dialkylanilin als säurebindendes Mittel und in Gegenwart einer katalytischen Menge des Hydrochlorids des betreffenden 2,6-Dialkylanilins bei Temperaturen von 100-250 °C durchführt. Doch auch mit diesem Verfahren liegen die erreichbaren Ausbeuten unter 70 % der Theorie.

Ferner wird in der schweizerischen Patentschrift 572,017 vorgeschlagenen, kernsubstituierte Aniline bei Temperaturen von 100-175 °C in Gegenwart eines tertiären Amins als säurebindendes Mittel mit α-Halogencarbonsäureestern umzusetzen, wobei gemäss einer bevorzugten Ausführungsform dieses Verfahrens die Umsetzung in überschüssigem Ester als Lösungsmittel durchgeführt und zur Beschleunigung der Reaktion das tertiäre Amin bereits zu Beginn der Umsetzung in Form eines Salzes, beispielsweise in Form des Hydrochlorids, zugesetzt wird. Nach diesem Verfahren werden Umsätze an Anilin von 90-96 %, eine Selektivität von 78-90 % und Ausbeuten an N-(1'-Alkoxycarbonylalkyl)-anilinen von 70-86 % der Theorie erreicht, wobei die Ausbeuteangaben nicht auf tatsächlich isoliertem Reinprodukt, sondern auf der analytischen Bestimmung des Gehalts an Reinprodukt im Rohprodukt beruhen.

Weiterhin ist aus der US-Patentschrift 4,008,066 bekannt, 2,6-dimethylanilin bei 120-125 °C in Gegenwart von Natriumhydrogencarbonat als säurebindendes Mittel mit dem 3-fach molaren Ueberschuss an 2-Brompropionsäuremethylester umzusetzen. Bei diesem Verfahren wird N-(1'-Methoxycarbonyläthyl)-2,6-dimethylanilin in einer Ausbeute von 79,6 % der Theorie erhalten.

Wie der vorstehende Ueberblick über den Stand der Technik zeigt, ist es mit den bisher bekannten Verfahren nicht möglich, N-(1'-Alkoxycarbonyläthyl)-2,6-dialkylaniline in befriedigender Ausbeute und Reinheit herzustellen. Es ist daher das Ziel der vorliegenden Erfindung, ein Verfahren vorzusehen, dass die Nachteile der bekannten Verfahren vermeidet und das die Herstellung von N-(1'-Alkoxycarbonyläthyl)-2,6-dialkylanilinen der Formel I in zufriedenstellender Ausbeute und Reinheit auf einfache Weise ermöglicht.

Gemäss vorliegender Erfindung wird vorgeschlagen, die N-(1'-Alkoxycarbonyläthyl)-2,6-dialkylaniline der Formel I in der Weise herzustellen, dass man einen 2-Chlorpropionsäureester der Formel II

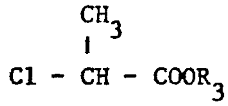

(II)

in welcher $R_3$ die unter Formel I angegebene Bedeutung hat, in Gegenwart von Wasser und einer quaternären Verbindung der Formel III

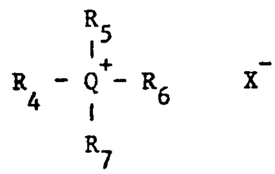

(III)

in welcher Q Stickstoff oder Phosphor darstellt, die Reste $R_4$, $R_5$, $R_6$ und $R_7$ je einen Alkylrest mit 1 bis 16 Kohlenstoffatomen oder Phenyl bedeuten und einer der Reste $R_4$, $R_5$, $R_6$ und $R_7$ auch für Benzyl stehen kann, wobei, wenn Q Stickstoff bedeutet, Q zusammen mit dreien der Reste $R_4$, $R_5$, $R_6$ und $R_7$ auch einen Pyridinrest bedeuten kann, während der vierte Alkyl mit 1-16 Kohlenstoffatomen, Phenyl oder Benzyl darstellt und $X^{\ominus}$ ein Halogenid- oder Bisulfatanion bedeutet, mit einem Alkalijodid zu einem Gemisch von 2-Chlorpropionsäureester und dem entsprechenden 2-Jodpropionsäureester umsetzt und dieses Estergemisch anschliessend nach Abtrennung der wässrigen Phase bei 100-130 °C in Gegenwart eines Alkalimetallcarbonats oder -hydrogencarbonats als säurebindendes Mittel mit überschüssigem 2,6-Dialkylanilin der Formel

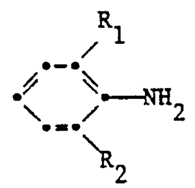

(IV)

in welcher $R_1$ und $R_2$ die unter Formel I angegebene Bedeutung haben, umsetzt, das Reaktionsgemisch mit Wasser extrahiert und das nach Abtrennung des wässrigen Extrakts erhaltene Gemisch aus N-(1'-Alkoxycarbonyläthyl)-2,6-dialkylanilin der Formel I und 2,6-dialkylanilin der Formel IV durch Destillation trennt.

Als Alkaljodide, die mit dem 2-Chlorpropionsäureester der Formel II zu einem Gemisch von 2-Chlorpropionsäureester und 2-Jodpropionsäureester umgesetzt werden, kommen Lithium-, Natrium-, Kalium-, Rubidium- und Cäsiumjodid in Betracht. Vorzugsweise werden Natrium- und Kaliumjodid verwendet. Das Alkalijodid kann in der Regel in einer Menge von 0,3-0,6 Mol pro Mol 2-Chlorpropionsäureester der Formel II eingesetzt werden, sodass ein Estergemisch erhalten wird das zu 40-70 Mol-% aus 2-Chlorpropionsäureester und zu 30-60 Mol-% aus 2-Jodpropionsäureester besteht. Vorzugsweise werden 0,45-0,55 Mol Alkalijodid pro Mol 2-Chlorpropionsäureester der Formel II verwendet, sodass ein Estergemisch resultiert, das etwa zu je 50 Mol-% aus 2-Chlorpropionsäureester und 2-Jodpropionsäureester besteht. Die Umsetzung wird in Gegenwart von Wasser durchgeführt. In der Regel verwendet man soviel Wasser, dass das Reaktionsgemisch zu etwa 40-50 Gewichtsprozent aus Wasser besteht.

Die quaternären Verbindungen der Formel III können erfindungsgemäss in einer Menge von 0,5-5 Gew.% bezogen auf eingesetzten 2-Chlorpropionsäureester der Formel II verwendet werden. Vorzugsweise verwendet man 1-3 Gew.% quaternäre Verbindungen der Formel III bezogen auf eingesetzten 2-Chlorpropionsäureester der Formel II.

Geeignete quaternäre Verbindungen der Formel III sind beispielsweise Tetrapropylammoniumchlorid, -bromid und -jodid, Tetrabutylammoniumchlorid, -bromid und -jodid, Tetrabutylammoniumbisulfat, Benzyltrumethylammoniumchlorid, Benzyltriäthylammoniumchlorid, Benzyltributylammoniumjodid, Tripropylammoniumjodid, Tripropylbutylammoniumbromid, Tributylmethylammoniumchlorid, Tributylmethylammoniumjodid, Trioctylmethylammoniumjodid, Trioctymethylammoniumbisulfat, Phenyltrimethylammoniumchlorid oder -bromid, Phenyltriäthylammoniumchlorid, Tricaprylmethylammoniumchlorid, Decyltrimethylammoniumchlorid, Dodecyltrimethylammoniumchlorid, Hexadecyltrimethylammoniumchlorid, Hexadecylpyridiniumchlorid, Dodecylpyridiniumbromid, Octylpyridiniumbromid, Tetrabutylphosphoniumchlorid, -bromid und -jodid, Benzyltrimethylphosphoniumchlorid, Benzyltributylphosphoniumchlorid, Tributylmethylphosphoniumchlorid, Trioctymethylphosphoniumbromid, Hexadecyltributylphosphoniumbromid, Decyltributylammoniumchlorid, Dodecyltriäthylammoniumbromid, Tetraphenylphosphoniumbromid, Hexadecyltrimethylphosphoniumbromid, Tributylmethylphosphoniumjodid und Tetraphenylphosphoniumbromid. Als besonders geeignete quaternäre Verbindung der Formel III ist Tetrabutylammoniumjodid zu nennen.

Das erfindungsgemäss im Ueberschuss zu verwendende 2,6-Dialkylanilin der Formel IV wird vorteilhaft in Mengen von 1,5-2,5 Mol pro Mol ursprünglich eingesetzten 2-Chlorpropionsäureester der Formel II verwendet. Die Verwendung eines geringeren oder grösseren Ueberschusses an 2,6-Dialkylanilin der Formel IV ist möglich, jedoch ist bei Verwendung von weniger als 1,5 Mol 2,6-Dialkylanilin der Formel IV pro Mol ursprünglich eingesetzten 2-Chlorpropionsäureester der Formel II das Reaktionsgemisch schwierig zu handhaben, während die Verwendung von mehr als 2,5 Mol 2,6-Dialkylanilin der Formel IV pro Mol ursprünglich eingesetzten 2-Chlorpropionsäureester der Formel II im Hinblick auf den mit der destillativen Entfernung des Ueberschüssigen 2,6-Dialkylanilins verbundenen Aufwand unwirtschaftlich ist. Vorzugsweise werden 1,6-1,8 Mol 2,6-Dialkylanilin der Formel IV pro Mol ursprünglich eingesetzten 2-Chlorpropionsäureester der Formel II verwendet.

Als säurebindende Mittel sind erfindungsgemäss Alkalicarbonate und Alkalihydrogencarbonate, wie Lithiumcarbonat, Natriumcarbonat, Kaliumcarbonat, Rubidiumcarbonat und Cäsiumcarbonat, sowie Lithiumhydrogencarbonat, Natriumhydrogencarbonat, Kaliumhydrogencarbonat, Rubidiumhydrogencarbonat und Cäsiumhydrogencarbonat geeignet. Bevorzugte säurebindende Mittel sind Natriumcarbonat, Kaliumcarbonat, Natriumhydrogencarbonat und Kaliumhydrogencarbonat. Die säurebindenden Mittel werden in der Regel in etwa stöchiometrischer Menge oder in einem Ueberschuss eingesetzt. Vorteilhaft werden die säurebindenden Mittel in einer Menge von 1-3 Aequivalenten bezogen auf den zu bindenden Halogenwasserstoff verwendet. Vorzugsweise verwendet man 1,1-1,3 Aequivalente säurebindendes Mittel bezogen auf den zu bindenden Halogenwasserstoff.

Innerhalb des angegebenen Temperaturbereichs von 100-130 °C, in dem die Umsetzung des 2,6-Dialkylanilins der Formel IV mit dem Gemisch aus den 2-Chlorpropionsäureester der Formel II und dem entsprechenden 2-Jodpropionsäureester durchgeführt wird, sind Temperaturen von 115-125 °C, insbesondere 115-120 °C, bevorzugt. Das während der Umsetzung eines 2,6-Dialkylanilins der Formel IV mit dem Gemisch aus 2-Chlorpropionsäureester der Formel II und dem entsprechenden 2-Jodpropionsäureester in Gegenwart eines Alkalicarbonats beziehungsweise -hydrogencarbonats gebildete Wasser wird vorteilhaft während der Umsetzung durch azeotrope Destillation getrennt. Die azeotrope Abtrennung des Wassers erfolgt vorteilhaft unter vermindertem Druck.

Nach beendigter Umsetzung wird das Reaktionsgemisch abgekühlt und durch Extraktion mit Wasser von Salzen befreit. Anschliessend wird aus der organischen Phase das überschüssige 2,6-Dialkylanilin der Formel IV durch Destillation getrennt. Die destillative Abtrennung des 2,6-Dialkylanilins der Formel IV erfolgt vorteilhaft im Vakuum. Das als Rückstand erhaltene N-(1'-Alkoxycarbonyläthyl)-2,6-dialkylanilin der Formel I kann in der Regel als solches für weitere Umsetzungen, beispielsweise für die eingangs genannte Umsetzung mit Carbonsäurechloriden, verwendet werden. Falls ein spezieller Anwendungszweck dies erforderlich macht, kann das Produkt jedoch auch durch eine Vakuum-Rektifikation gereinigt werden.

Der nach der Extraktion des Reaktionsgemisches mit Wasser erhaltene wässrige Extrakt, der das gesamte ursprünglich eingesetzte Alkalijodid enthält, wird erfindungsgemäss nach Zugabe der entsprechenden Menge an quaternärer Verbindung der Formel III mit frischem 2-Chlorpropionsäureester der Formel II umgesetzt. Auf diese Weise können Jodverluste im wesentlichen vermieden werden.

Gemäss einer bevorzugten Ausführungsform des erfindungsgemässen Verfahrens setzt man einen 2-Chlorpropionsäureester der Formel II in Gegenwart von Wasser und 1-3 Gew.-% Tetrabutylammoniumjodid bezogen auf eingesetzten 2-Chlorpropionsäureester der Formel II mit 0,45-0,55 Mol Natrium- oder Kaliumjodid pro Mol 2-Chlorpropionsäureester der Formel II zu einem Gemisch von 2-Chlorpropionsäureester der Formel II und dem entsprechenden 2-Jodpropionsäureester um und lässt dieses Gemisch nach Abtrennung der wässrigen Phase bei 115-125 °C, insbesondere 115-120 °C, in Gegenwart von 0,55 Mol Natriumcarbonat pro Mol ursprünglich eingesetzten 2-Chlorpropionsäureester der Formel II mit 1,7 Mol 2,6-Dialkylanilin der Formel IV pro Mol ursprünglich eingesetzten 2-Chlorpropionsäureester der Formel II reagieren, extrahiert das Reaktionsgemisch mit Wasser und trennt das nach Abtrennung des wässrigen Extrakts erhaltene Gemisch aus 2,6-Dialkylanilin der Formel IV und N-(1'-Alkoxycarbonyläthyl)-2,6-dialkylanilin der Formel I durch Destillation. Nach dieser bevorzugten Ausführungsform lassen sich insbesondere 2,6-Dimethylanilin und 2-Chlorpropionsäuremethylester vorteilhaft umsetzen.

Mit dem erfindungsgemässen Verfahren wird es möglich, die N-(1'-Alkoxycarbonyläthyl)-2,6-dialkylaniline der Formel I in höheren Ausbeuten und in besserer Qualität herzustellen als nach den bisher bekannten Verfahren. Das erfindungsgemässe Verfahren kann auf einfache Weise in üblichen Apparaturen durchgeführt werden und eignet sich insbesondere für die kontinuierliche Durchführung. Im Hinblick auf die hohe Ausbeute und Qualität der Verfahrensprodukte, den geringen apparativen Aufwand und die Tatsache, dass Verluste an teurem Jodid durch Wiederverwendung des als Nebenprodukt erhaltenen Alkalijodids auf ein Minimum beschränkt werden können, kann das erfindungsgemässe Verfahren als besonders wirtschaftlich angesehen werden. Das erfindungsgemässe Verfahren bietet gegenüber bekannten Verfahren auch ökologische Vorteile, da wegen der hohen Selektivität und der hohen Ausbeute nur sehr geringe Mengen an Neben- und Zersetzungsprodukten ins Abwasser gelangen.

Das erfindungsgemässe Verfahren wird durch das folgende Beispiel näher erläutert.

## Beispiel

Herstellung von N-(1'-Methoxycarbonyläthyl)-2,6-dimethylanilin.

Eine Mischung von 180,0 g (1,2 Mol) Natriumjodid, 245,0 g (2,0 Mol) 2-Chlorpropionsäuremethylester, 5,0 g Tetrabutylammoniumjodid und 400 g Wasser wird 3 Stunden bei Rückflusstemperatur gerührt. Nach Abkühlen auf Raumtemperatur und Phasentrennung erhält man 320 g organische Phase

und 489 g wässrige Phase. Die organische Phase, die 45 Mol-% 2-Chlorpropionsäuremethylester und 55 Mol-% 2-Jodpropionsäuremethylester sowie das Tetrabutylammoniumjodid enthält, wird nach Zugabe von 411,0 g (3,4 Mol) 2,6-Dimethylanilin und 115,0 g (1,08 Mol) Natriumcarbonat unter Rühren auf 105 °C aufgeheizt und 7 Stunden bei dieser Temperatur gehalten. Dann wird das Reaktionsgemisch auf 25 °C abgekühlt und mit 400 g Wasser extrahiert. Der erhaltene wässrige Extrakt wird nach Zugabe von 5,0 g Tetrabutylammoniumjodid zur Umsetzung mit weiteren 2-Chlorpropionsäuremethylester verwendet. Die organische Phase (580 g) wird durch Destillation aufgearbeitet. Man erhält 190,0 g 2,6-Dimethylanilin und 362,0 g (95,7 % der Theorie bezogen auf umgesetztes 2,6-Dimethylanilin) N-(1'-Methoxycarbonyläthyl-2,6-dimethylanilin).

**Ansprüche**

1. Verfahren zur Herstellung von N-(1'-Alkoxycarbonyläthyl)-2,6-dialkylanilinen der Formel I

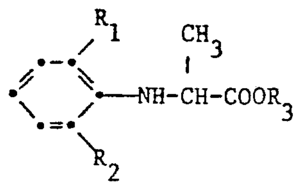

(I)

in welcher $R_1$ und $R_2$ je Methyl oder Aethyl bedeuten und $R_3$ für eine Alkylgruppe von 1-4 Kohlenstoffatomen steht, dadurch gekennzeichnet, dass man einen 2-Chlorpropionsäureester der Formel II

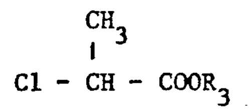

(II)

in welcher $R_3$ die oben angegebene Bedeutung hat, in Gegenwart von Wasser und einer quaternären Verbindung der Formel III

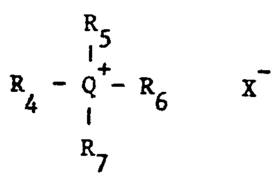

(III)

in welcher Q Stickstoff oder Phosphor darstellt, die Reste $R_4$, $R_5$, $R_6$ und $R_7$ je einen Alkylrest mit 1 bis 16 Kohlenstoffatomen oder Phenyl bedeuten und einer der Reste $R_4$, $R_5$, $R_6$ und $R_7$ auch für Benzyl stehen kann, wobei, wenn Q Stickstoff bedeutet, Q zusammen mit dreien der Reste $R_4$, $R_5$, $R_6$ und $R_7$ auch einen Pyridinrest bedeuten kann, während der vierte Alkyl mit 1-16 Kohlenstoffatomen, Phenyl oder Benzyl darstellt und $X^{\ominus}$ ein Halogenid- oder Bisulfitanion darstellt, mit einem Alkalijodid zu einem Gemisch von 2-Chlorpropionsäureester der Formel II und dem entsprechenden 2-Jodpropionsäureester umsetzt, dieses Gemisch anschliessend nach Abtrennung der wässrigen Phase bei 100-130 °C in Gegenwart eines Alkalimetallcarbonats- oder Hydrogencarbonats als säurebindendes Mittel mit überschüssigem 2,6-Dialkylanilin der Formel IV

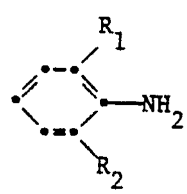

(IV)

in welcher $R_1$ und $R_2$ die oben angegebene Bedeutung haben, umsetzt, das Reaktionsgemisch mit

Wasser extrahiert und das nach Abtrennung des wässrigen Extrakts erhaltene Gemisch von 2,6-Dialkylanilin der Formel IV und N-(1'-Alkoxycarbonyläthyl)-2,6-dialkylanilin der Formel I durch Destillation trennt.

2. Verfahren nach Anspruch 1, dadurch gekennzeichnet, dass man das Alkalijodid in einer Menge von 0,3-0,6 Mol pro Mol 2-Chlorpropionsäureester der Formel II einsetzt.

3. Verfahren nach Anspruch 1, dadurch gekennzeichnet, dass man das Alkalijodid in einer Menge von 0,45-0,55 Mol pro Mol 2-Chlorpropionsäureester der Formel II einsetzt.

4. Verfahren nach Anspruch 1, dadurch gekennzeichnet, dass man als Alkalijodid Natriumjodid oder Kaliumjodid verwendet.

5. Verfahren nach Anspruch 1, dadurch gekennzeichnet, dass man die quaternäre Verbindung der Formel III in einer Menge von 0,5-5,0 Gew.-% bezogen auf eingesetzten 2-Chlorpropionsäureester der Formel II verwendet.

6. Verfahren nach Anspruch 1, dadurch gekennzeichnet, dass man die quaternäre Verbindung der Formel II in einer Menge von 1-3 Gew.-% bezogen auf eingesetzten 2-Chlorpropionsäureester der Formel II verwendet.

7. Verfahren nach Anspruch 1, dadurch gekennzeichnet, dass man als quaternäre Verbindung der Formel III Tetrabutylammoniumjodid verwendet.

8. Verfahren nach Anspruch 1, dadurch gekennzeichnet, dass man das 2,6-Dialkylanilin der Formel IV in Mengen von 1,5-2,5 Mol pro Mol ursprünglich eingesetzten 2-Chlorpropionsäureester verwendet.

9. Verfahren nach Anspruch 1, dadurch gekennzeichnet, dass man das 2,6-Dialkylanilin der Formel IV in einer Menge von 1,6-1,8 Mol pro Mol ursprünglich eingesetzten 2-Chlorpropionsäureester verwendet.

10. Verfahren nach Anspruch 1, dadurch gekennzeichnet, dass man das säurebindende Mittel in einer Menge von 1-3 Aequivalenten bezogen auf den zu bindenden Halogenwasserstoff verwendet.

11. Verfahren nach Anspruch 1, dadurch gekennzeichnet, dass man 1,1-1,3 Aequivalente säurebindendes Mittel bezogen auf den zu bindenden Halogenwasserstoff verwendet.

12. Verfahren nach Anspruch 1, dadurch gekennzeichnet, dass man die Umsetzung eines 2,6-Dialkylanilins der Formel IV mit dem Gemisch von 2-Chlorpropionsäureester der Formel II und dem entsprechenden 2-Jodpropionsäureester bei einer Temperatur von 115-120 °C durchführt.

13. Verfahren nach Anspruch 1, dadurch gekennzeichnet, dass man den nach Umsetzung eines Gemisches von 2-Chlorpropionsäureester der Formel II und dem entsprechenden 2-Jodpropionsäureester mit einem 2,6-Dialkylanilin der Formel IV und Extraktion des Reaktionsgemisches mit Wasser erhaltenen wässrigen Extrakt nach Zugabe einer quaternären Verbindung der Formel III zur Umsetzung mit einem 2-Chlorpropionsäureester der Formel II verwendet.

14. Verfahren nach Anspruch 1, dadurch gekennzeichnet, dass man eine 2-Chlorpropionsäureester der Formel II in Gegenwart von Wasser und 1-3 Gew.-% Tetrabutylammoniumjodid bezogen auf eingesetzten 2-Chlorpropionsäureester der Formel II mit 0,45-0,55 Mol Natrium- oder Kaliumjodid pro Mol 2-Chlorpropionsäureester der Formel II zu einem Gemisch von 2-Chlorpropionsäureester der Formel II und dem entsprechenden 2-Jodpropionsäureester umsetzt und dieses Gemisch nach Abtrennung der wässrigen Phase bei 115-120 °C in Gegenwart von 0,55 Mol Natriumcarbonat pro Mol ursprünglich eingesetzten 2-Chlorpropionsäureester der Formel II mit 1,7 Mol 2,6-Dialkylanilin der Formel IV pro Mol ursprünglich eingesetzten 2-Chlorpropionsäureester der Formel II reagieren lässt, das Reaktionsgemisch mit Wasser extrahiert und das nach der Abtrennung des wässrigen Extrakts erhaltene Gemisch aus 2,6-Dialkylanilin der Formel IV und N-(1'-Alkoxycarbonyläthyl)-2,6-dialkylanilin der Formel I durch Destillation trennt.

15. Verfahren nach Anspruch 1, dadurch gekennzeichnet, dass man als 2,6-Dialkylanilin der Formel IV 2,6-Dimethylanilin verwendet.

16. Verfahren nach Anspruch 1, dadurch gekennzeichnet, dass man als 2-Chlorpropionsäureester der Formel II 2-Chlorpropionsäuremethylester verwendet.

## Claims

1. A process for the preparation of N-(1'-alkoxycarbonylethyl)-2,6-dialkylanilines of the formula I

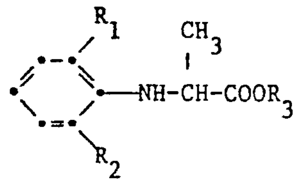

(I)

in which $R_1$ and $R_2$ are each methyl or ethyl and $R_3$ is an alkyl group having 1-4 carbon atoms, which comprises reacting a 2-chloropropionic acid ester of the formula II

$$\begin{array}{c} \quad CH_3 \\ \quad | \\ Cl - CH - COOR_3 \end{array} \qquad (II)$$

in which $R_3$ is as defined above, in the presence of water and of a quaternary compound of the formula III

$$\begin{array}{c} R_5 \\ | \\ R_4 - \overset{+}{Q} - R_6 \\ | \\ R_7 \end{array} \quad X^- \qquad (III)$$

in which Q is nitrogen or phosphorus, the radicals $R_4$, $R_5$, $R_6$ and $R_7$ are each an alkyl radical having 1 to 16 carbon atoms, or phenyl, and one of the radicals $R_4$, $R_5$, $R_6$ and $R_7$ can also be benzyl, and, if Q is nitrogen, Q together with three of the radicals $R_4$, $R_5$, $R_6$ and $R_7$ can also be a pyridine radical, whilst the fourth radical is alkyl having 1-16 carbon atoms, phenyl or benzyl, and $X^{\ominus}$ is a halide anion or a bisulfate anion, with an alkali metal iodide to give a mixture of the 2-chloropropionic acid ester of the formula II and the corresponding 2-iodopropionic acid ester, subsequently reacting this mixture, after separating off the aqueous phase, at 100-130 °C, in the presence of an alkali metal carbonate or alkali metal bicarbonate as an acid acceptor, with excess 2,6-dialkylaniline of the formula IV

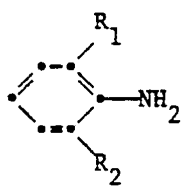

(IV)

in which $R_1$ and $R_2$ are as defined, extracting the reaction mixture with water and separating, by distillation, the mixture of 2,6-dialkylaniline of the formula IV and N-(1'-alkoxycarbonylethyl)-2,6-dialkylaniline of the formula I, which is obtained after separating off the aqueous extract.

2. A process according to claim 1, wherein the alkali metal iodide is employed in an amount of 0,3-0,6 mol per mol of 2-chloropropionic acid ester of the formula II.

3. A process according to claim 1, wherein the alkali metal iodide is employed in an amount of 0,45-0,55 mol per mol of 2-chloropropionic acid ester of the formula .II.

4. A process according to claim 1, wherein the alkali metal iodide used is sodium iodide or potassium iodide.

5. A process according to claim 1, wherein the quaternary compound of the formula III is used in an amount of 0,5-5,0 % by weight, based on the 2-chloropropionic acid ester of the formula II employed.

6. A process according to claim 1, wherein the quaternary compound of the formula III is used in an amount of 1-3 % by weight, based on the 2-chloropropionic acid ester of the formula II employed.

7. A process according to claim 1, wherein the quaternary compound of the formula III which is used is tetrabutylammonium iodide.

8. A process according to claim 1, wherein the 2,6-dialkylaniline of the formula IV is used in amounts of 1,5-2,5 mols per mol of 2-chloropropionic acid ester originally employed.

9. A process according to claim 1, wherein the 2,6-dialkylaniline of the formula IV is used in an amount of 1,6-1,8 mols per mol of 2-chloropropionic acid ester originally employed.

10. A process according to claim 1, wherein the acid-binding agent is used in an amount of 1-3 equivalents, based on the hydrogen halide to be bonded.

11. A process according to claim 1, wherein 1,1-1,3 equivalents of acid-binding agent are used, based on the hydrogen halide to be bonded.

12. A process according to claim 1, wherein the reaction of a 2,6-dialkylaniline of the formula IV with the mixture of the 2-chloropropionic acid ester of the formula II and the corresponding 2-iodopropionic acid ester is carried out at a temperature of 115-120 °C.

13. A process according to claim 1, wherein the aqueous extract obtained after reacting a mixture of the 2-chloropropionic acid ester of the formula II and the corresponding 2-iodopropionic acid ester with a 2,6-dialkylaniline of the formula IV and extracting the reaction mixture with water is used, after the addition of a quaternary compound of the formula III, for reaction with a 2-chloropropionic acid ester of the formula II.

14. A process according to claim 1, wherein a 2-chloropropionic acid ester of the formula II is reacted in the presence of water and 1-3 % by weight of tetrabutylammonium iodide, based on 2-chloropropionic

acid ester of the formula II employed, with 0,45-0,55 mol of sodium iodide or potassium iodide per mol of 2-chloropropionic acid ester of the formula II to give a mixture of the 2-chloropropionic acid ester of the formula II and the corresponding 2-iodopropionic acid ester, and, after separating off the aqueous phase, this mixture is allowed to react, at 115-120 °C, in the presence of 0,55 mol of sodium carbonate per mol of 2-chloropropionic acid ester of the formula II originally employed, with 1,7 mols of a 2,6-dialkylaniline of the formula IV, per mol of 2-chloropropionic acid ester of the formula II originally employed, the reaction mixture is extracted with water and the mixture of the 2,6-dialkylaniline of the formula IV and the N-(1'-alkoxycarbonylethyl)-2,6-dialkylaniline of the formula I, which is obtained after separating off the aqueous extract, is separated by distillation.

15. A process according to claim 1, wherein the 2,6-dialkylaniline of the formula IV which is used is 2,6-dimethylaniline.

16. A process according to claim 1, wherein the 2-chloropropionic acid ester of the formula II which is used is methyl 2-chloropropionate.

**Revendications**

1. Procédé de préparation de N-(1'-alcoxycarbonyléthyl)-2,6-dialkylanilines de formule I

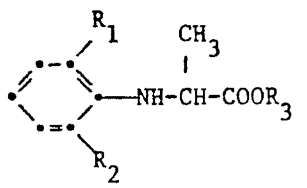

(I)

dans laquelle $R_1$ et $R_2$ représentent chacun un groupe méthyle ou éthyle et $R_3$ représente un groupe alkyle en C1-C4, caractérisé en ce que l'on fait réagir un ester 2-chloropropionique de formule II

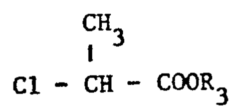

(II)

dans laquelle $R_3$ a la signification indiquée ci-dessus, en présence d'eau et d'un composé quaternaire de formule III

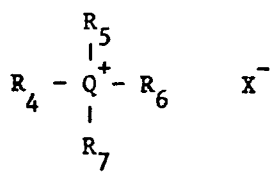

(III)

dans laquelle Q représente l'azote ou le phosphore, les symboles $R_4$, $R_5$, $R_6$ et $R_7$ représentent chacun un groupe alkyle en C1-C16 ou phényle, l'un des symboles $R_4$, $R_5$, $R_6$ et $R_7$ pouvant également représenter un groupe benzyle, Q, lorsqu'il représente l'azote, pouvant également former un reste de pyridine avec trois des restes $R_4$, $R_5$, $R_6$ et $R_7$, le quatrième étant un groupe alkyle en C1-C16, phényle ou benzyle, et $X^{\ominus}$ représente un anion halogénure ou bisulfite, avec un iodure alcalin, la réaction donnant un mélange de l'ester 2-chloropropionique de formule II et de l'ester 2-iodopropionique correspondant qu'on fait ensuite réagir, après séparation de la phase aqueuse, à une température de 100 à 130 °C, en présence d'un carbonate ou bicarbonate de métal alcalin qui sert d'agent fixant les acides, avec un excès d'une 2,6-dialkylaniline de formule IV

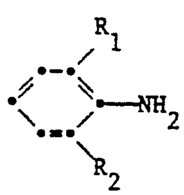

(IV)

dans laquelle $R_1$ et $R_2$ ont les significations indiquées ci-dessus, on extrait le mélange de réaction par l'eau et on sépare par distillation le mélange de 2,6-dialkylaniline de formule IV et de N-(1'-alcoxycarbo-

nyléthyl)-2,6-dialkylaniline de formule I obtenu après séparation de l'extrait aqueux.

2. Procédé selon la revendication 1, caractérisé en ce que l'on utilise l'iodure alcalin en quantité de 0,3 à 0,6 mole par mole de l'ester 2-chloropropionique de formule II.

3. Procédé selon la revendication 1, caractérisé en ce que l'on utilise l'iodure alcalin en quantité de 0,45 à 0,55 mole par mole de l'ester 2-chloropropionique de formule II.

4. Procédé selon la revendication 1, caractérisé en ce que l'on utilise en tant qu'iodure alcalin l'iodure de sodium ou l'iodure de potassium.

5. Procédé selon la revendication 1, caractérisé en ce que l'on utilise le composé quaternaire de formule III en quantité de 0,5 à 5,0 % en poids par rapport à l'ester 2-chloropropionique de formule II mis en œuvre.

6. Procédé selon la revendication 1, caractérisé en ce que l'on utilise le composé quaternaire de formule III en quantité de 1 à 3 % en poids par rapport à l'ester 2-chloropropionique de formule II mis en œuvre.

7. Procédé selon la revendication 1, caractérisé en ce que l'on utilise en tant que composé quaternaire de formule III l'iodure de tétrabutylammonium.

8. Procédé selon la revendication 1, caractérisé en ce que l'on utilise la 2,6-dialkylaniline de formule IV en quantité de 1,5 à 2,5 moles par mole de l'ester 2-chloropropionique mise en œuvre à l'origine.

9. Procédé selon la revendication 1, caractérisé en ce que l'on utilise la 2,6-dialkylaniline de formule IV en quantité de 1,6 à 1,8 mole par mole de l'ester 2-chloropropionique mise en œuvre à l'origine.

10. Procédé selon la revendication 1, caractérisé en ce que l'on utilise l'agent fixant les acides en quantité de 1 à 3 équivalents par rapport à l'halogénure d'hydrogène à fixer.

11. Procédé selon la revendication 1, caractérisé en ce que l'on utilise de 1,1 à 1,3 équivalent de l'agent fixant les acides par rapport à l'halogénure d'hydrogène à fixer.

12. Procédé selon la revendication 1, caractérisé en ce que l'on effectue la réaction entre une 2,6-dialkylaniline de formule IV et le mélange de l'ester 2-chloropropionique de formule II et de l'ester 2-iodopropionique correspondant à une température de 115 à 120 °C.

13. Procédé selon la revendication 1, caractérisé en ce que, après réaction d'un mélange de l'ester 2-chloropropionique de formule II et de l'ester 2-iodopropionique correspondant avec une 2,6-dialkylaniline de formule IV et extraction du mélange de réaction par l'eau, on utilise l'extrait aqueux obtenu, après addition d'un composé quaternaire de formule III, pour la réaction avec un ester 2-chloropropionique de formule II.

14. Procédé selon la revendication 1, caractérisé en ce que l'on fait réagir un ester 2-chloropropionique de formule II en présence d'eau et de 1 à 3 % en poids d'iodure de tétrabutylammonium par rapport à l'ester 2-chloropropionique de formule II mis en œuvre avec 0,45 à 0,55 mole d'iodure de sodium ou de potassium par mole de l'ester 2-chloropropionique de formule II, la réaction donnant un mélange de l'ester 2-chloropropionique de formule II et de l'ester 2-iodopropionique correspondant qu'on fait réagir, après séparation de la phase aqueuse, à 115-120 °C, en présence de 0,55 mole de carbonate de sodium par mole de l'ester 2-chloropropionique de formule II mise en œuvre à l'origine, avec 1,7 mole d'une 2,6-dialkylaniline de formule IV par mole de l'ester 2-chloropropionique de formule II mise en œuvre à l'origine, on extrait le mélange de réaction par l'eau et on sépare par distillation le mélange d'une 2,6-dialkylaniline de formule IV et d'une N-(1'-alcoxycarbonyléthyl)-2,6-dialkylaniline de formule I obtenu après séparation de l'extrait aqueux.

15. Procédé selon la revendication 1, caractérisé en ce que l'on utilise en tant que 2,6-dialkylaniline de formule IV la 2,6-diméthylaniline.

16. Procédé selon la revendication 1, caractérisé en ce que l'on utilise en tant qu'ester 2-chloropropionique de formule II le 2-chloropropionate de méthyle.